# EUROPÄISCHE PATENTSCHRIFT

(11) **EP 0 665 428 B1**
(45) Veröffentlichungstag und Bekanntmachung des Hinweises auf die Patenterteilung: **28.11.2001**
(21) Anmeldenummer: 94118637.1
(22) Anmeldetag: 26.11.1994
(51) Int. Cl.: G01N 27/12, G01N 33/00

(54) **Einrichtung für die Messung von Zustandsgrössen in Gasen mit zumindest einem Halbleiter-Gassensor**
Device for measuring parameters of gases using at least one semi conductor gas-sensor
Dispositif de mesure de paramètres de gaz utilisant au moins un capteur de gaz semi-conducteur

(30) Priorität: 20.01.1994 DE 4401570
(43) Veröffentlichungstag der Anmeldung: 02.08.1995
(73) Patentinhaber: RWE Rheinbraun Aktiengesellschaft, 50935 Köln (DE)
(72) Erfinder: Kelleter Jörg, Dipl.Phys. Justus-Liebig-Universit., D-35392 Giessen (DE); Kohl Claus-Dieter, Prof. Justus-Liebig-Universität, D-35392 Giessen (DE); Petig Heinz, Dipl.-Ing., D-45277 Essen (DE)
(74) Vertreter: Honke, Manfred, Dr.-Ing.

(56) Entgegenhaltungen:
- EP-A- 0 527 258
- EP-A- 0 571 783
- DE-A- 3 809 107
- DE-U- 9 113 607
- DE-U- 9 309 638
- DE-U- 9 309 640

## Beschreibung

Die Erfindung betrifft eine Einrichtung für die Messung von Zustandsgrößen in Gasen mit zumindest einem Halbleiter-Gassensor in einer Schaltungsanordnung mit Netzteil und Meßsignalaufnehmer. Der Ausdruck Messung umfaßt quantitative Messungen und/oder qualitative und/oder identifizierende Messungen und/oder Überwachungen. Die Zustandsgrößen eines Gases meint die thermodynamischen Zustandsgrößen, bei Gasmischungen auch die Konzentrationen der Mischungskomponenten. Insoweit kann die Einrichtung auch als Detektor für bestimmte Gase in einer Gasmischung arbeiten.

Einrichtungen des beschriebenen Aufbaus und der beschriebenen Zweckbestimmung sind in verschiedenen Ausführungsfornen bekannt. Sie können im einzelnen unterschiedlich aufgebaut und gestaltet und dadurch unterschiedlichen Anwendungen angepaßt sein (vgl. G 91 13 607.5, G 93 09 638.0, G 93 09 640.2). Die Auswertung der Meßsignale kann auch dadurch erfolgen, daß die Temperatur oder andere Zustandsgrößen der Gase durch einen Vergleich von bei mindestens zwei unterschiedlichen Zuständen des Halbleiter-Gassensors gemessen und aus den Messungen Gaszustands- und Entwicklungsmuster entwickelt und mit gespeicherten Mustern verglichen werden. Die unterschiedlichen Zustände der Halbleiter-Gassensoren werden vorzugsweise an einem Halbleiter-Gassensor zyklisch erzeugt oder die Auswertung wird zyklisch geändert. Der Vergleich kann anhand von Zeitverlaufsähnlichkeiten, insbes. nach Filterung der Ausgangssignale der Halbleiter-Gassensoren, z. B. über Tiefpaßfilterung, erfolgen. Die Vergleichsmuster wird man bei einer derartigen Auswertung in Abhängigkeit vom Meßort, vorzugsweise am jeweiligen Meßort selbst, ermitteln. In den Mustern bilden im allgemeinen die Zusammensetzungsverhältnisse und ihre Quotienten die Hauptbestandteile (vgl. DE 43 02 367.3 A1). Die Einrichtung kann eine elektrische, die Gasverhältnisse angebende Signalbildungseinheit aufweisen, die z. B. die Einzelkonzentrationen von oxidierbaren Gasen, ihre Quotienten und insbes. deren Veränderungsguotienten ermittelt und ausgibt, wobei die Halbleiter-Gassensoren eine Gasbeeinflussungsdeckschicht aufweisen können, die ihre Oberfläche ganz oder teilweise bedeckt und insbes. die Diffusion der oxidierbaren sowie weiteren Gase zum Metalloxid gezielt beeinflußt (DE 43 21 736.2 A1). Zur Physik der Zusammenhänge beim Betrieb einer solchen Einrichtung in bezug auf die Oberflächenphänomene der Halbleiter-Gassensoren wird z B. auf phys. stat. sol. (a) 49, 27 ff. (1978) verwiesen. Gebräuchliche Halbleiter-Gassensoren basieren auf den Oxiden SnO₂, WO₃, Fe₂O₃, In₂O₃ oder auf organischen Verbindungen wie z. B. den Phthalocyaninen oder Pyrolen. Ihre Auswahl für den Einsatz in einer Einrichtung des beschriebenen grundsätzlichen Aufbaus erfolgt nach Maßgabe spezieller Zweckbestimmungen unter Berücksichtigung der bekannten Charakteristiken der Halbleiter-Gassensoren.

Bei dem bekannten Gerät, von dem die Erfindung ausgeht, weist der Halbleiter-Gassensor eine Isolierschicht und auf einer Seite der Isolierschicht eine Leiterstruktur sowie darauf eine aktive Halbleiterschicht auf, in die zwei Elektroden mit Abstand voneinander eingebettet sind. Über die Leiterstruktur, die einem Heizkreis angehört, kann die Betriebstemperatur des Fialbleiter-Gassenors eingestellt werden. Es ist bekannt, dabei mit gepulsten oder nicht gepulsten Betriebsspannungen sowie gepulsten oder nicht gepulsten Meßspannungen zu arbeiten und dazu einen oder mehrere Pulsspannungs-Generatoren in die Schaltungsanordnung zu integrieren.

Bei der bekannten Einrichtung, von der die Erfindung ausgeht, ist die Langzeitstabilität der Halbleiter-Gassensoren, insbesondere in bezug auf die Lage des Nullpunktes, starken Schwankungen und Streuungen unterworfen. Die Langzeitstabilität hängt von den Herstellungsmaßnahmen sowie den konkreten Betriebsbedingungen ab und wird beeinflußt durch die Wahl der Arbeitstemperatur, durch die Häufigkeit von Temperaturwechseln und durch die Gaskomponenten sowie deren Konzentrationsschwankungen, denen ein Halbleiter-Gassensor ausgesetzt ist. Die Nachweisempfindlichkeit bei Halbleitersensoren verändert sich z. B. durch Änderung der Morphologie, durch Änderungen bei den katalytisch aktiven Zusätzen oder durch irreversible Adsorption von Gasen, die sehr feste Bindungen ausbilden. Diese Einflüsse können einen Halbleiter-Gassensor nach mehr oder weniger kurzer Betriebszeit unbrauchbar machen. Insoweit ist bei bekannten Einrichtungen die Betriebssicherheit der Halbleiter-Gassensoren verbesserungsbedürftig. Um Ausfälle zu vermeiden, behilft man sich bisher durch sog. Nachkalibrieren der Halbleiter-Gassensoren mit Prüfgas in mehr oder weniger kurzen zeitlichen Abständen. Das ist aufwendig.

Der Erfindung liegt das technische Problem zugrunde, bei einer Einrichtung für die Messung von Zustandsgrößen in Gasen mit zumindest einem Halbleiter-Gassensor die Funktionssicherheit unter Verzicht von Nachkalibrierungsmaßnahmen zu verbessern.

Zur Lösung dieses technischen Problems ist Gegenstand der Erfindung eine Einrichtung für die Messung von Zustandsgrößen in Gasen mit zumindest einem Halbleiter-Gassensor in einer Schaltungsanordnung mit Netzteil und Meßsignalaufnehmer - mit den folgenden Merkmalen:
a) der Halbleiter-Gassensor weist zwei mit Abstand voneinander angeordnete Elektroden sowie eine Isolierschicht und auf der Isolierschicht eine Feldelektrode auf,
b) die beiden Elektroden sind in eine sensor aktive Halbleiterschicht eingebettet, die Feldelektrode ist gegen die Halbleiterschicht und gegen die Elektroden elektrisch isoliert,
c) ein Pulsspannungs-Generator ist einerseits an die Feldelektrode und andererseits an eine der in die Halbleiterschicht eingebetteten Elektroden angeschlossen,
wobei zur Messung der Zustandsgrößen der Leitwert der aktiven Halbleiterschicht über die Elektroden gemessen wird und außerdem zur autogenen Funktionskontrolle des Halbleiter-Gassensors in zeitlichen Abständen ein Puls des Pulsspannungs-Generators zwischen Feldelektrode und eine der in die aktive Halbleiterschicht eingebetteten Elektroden angelegt wird und die durch das vom Spannungspuls erzeugte elektrische Feld verursachte Leitwertänderung der Halbleiterschicht als Funktion der Zeit über den Meßsignalaufnehmer gemessen wird.

Die Erfindung geht von der Erkenntnis aus, daß eine Kontrolle der Funktion einer erfindungsgemäßen Einrichtung möglich ist, wenn zusätzlich eine Feldelektrode wie beschrieben angeordnet und der Halbleiter-Gassensor wie beschrieben betrieben wird. Es kann ohne weiteres neben der üblichen Leitwertmessung zum Zwecke der Erfassung der Zustandsgrößen eine autogene Funktionskontrolle des Halbleiter-Gassensors in zeitlichen Abständen durchgeführt werden, und zwar durch die beschriebene Pulsspannungsüberlagerung. Die Einzelheiten werden im Ausführungsbeispiel erläutert.

Im einzelnen bestehen im Rahmen der Erfindung mehrere Möglichkeiten der weiteren Ausbildung und Gestaltung. Bewährt hat sich eine Ausführungsform, bei der der Halbleiter-Gassensor auf einer Seite der Isolierschicht die beiden Elektroden sowie die aktive Halbleiterschicht und auf der anderen Seite der Isolierschicht die Feldelektrode aufweist. Bewährt hat sich außerdem eine Ausführungsform, bei der der Halbleiter-Gassensor auf einer Seite der Isolierschicht die beiden Elektroden und, getrennt von diesen, die Feldelektrode aufweist, die durch eine abdeckende Glasisolierschicht von der aktiven Halbleiterschicht, in die die Elektroden eingebettet sind, getrennt ist, wobei zusätzlich auf der Isolierschicht eine Leiterstruktur angeordnet ist, die einem Heizkreis angehört.

Als Isolierschicht kann eine Dünnschicht mit einer Dicke von 30 bis 100 nm eingesetzt werden, die beispielsweise aus Siliziumoxid und/oder Siliziumnitrid und/oder aus Aluminiumoxid besteht. Die Leiterstruktur besteht zweckmäßigerweise aus einem gut leitenden Metall oder aus Polysilizium. Eei der erfindungsgemäßen Einrichtung empfiehlt es sich, dafür zu sorgen, daß die Feldelektrode mit der verfügbaren Betriebsspannung möglichst große Feldstärken erzeugen kann. Dazu lehrt die Erfindung, die Auslegung insgesamt so zu treffen, daß die Feldelektrode elektrische Feldstärken in der Größenordnung von 10⁴ bis 10⁶ V/cm erzeugt. Das Netzteil kann für eine Betriebsspannung von etwa 10 V ausgelegt sein, es kann aber auch mit geringerer Spannung betrieben werden.

Zur Steuerung des Ablaufes der Meßvorgänge und zur Auswertung der Meßergebnisse weist zweckmäßigerweise die Schaltungsanordnung einen Microcontroller auf und steuert dieser den Ablauf der Meßvorgänge. Der Microcontroller kann fernerhin sowohl bei der Leitwertmessung im Rahmen der Messung der Zustandsgrößen als auch bei der autogenen Funktionskontrolle des Halbleiter-Gassensors als Auswerter eingesetzt werden. Im Rahmen der Erfindung liegt es, die Einrichtung so zu gestalten, daß zur Einstellung von kritischen Meßbereichen des aktiven Halbleiter-Gassensors dessen Betriebstemperatur über die Leiterstrukur, an die ein Heizkreis angeschlossen ist, veränderbar ist. Dazu kann der Betrieb des aktiven Halbleiter-Gassensors mit Gleichspannung erfolgen, es besteht aber auch die Möglichkeit, den Betrieb des aktiven Halbleiter-Gassensors mit gepulster Gleichspannung durchzuführen.

Zur Theorie der Zusammenhänge wird folgendes vorgetragen: Die nachzuweisenden Gase erzeugen entweder Donatoren oder Akzeptoren, die die Ladungsträgerdichte in der aktiven Halbleiterschicht erhöhen oder absenken. Die dadurch verursachte Leitwertänderung dient als Sensorsignal, aus dem die Gaskonzentration bestimmt wird. An jeder Oberfläche der Halbleiterschicht wird jedoch ein Teil dieser Ladungen in sog. Haftstellen festgelegt. Durch das starke elektrische Feld, welches bei der erfindungsgemäßen Einrichtung mit Hilfe der Feldelektrode erzeugt wird, ändert sich die Ladungsdichte in den Haftstellen und in der Halbleiterschicht. Nach Abschalten des Feldes stellt sich die ursprüngliche Ladungsverteilung wieder ein. Aufgrund der Morphologie der Oberfläche und wegen irreversibel gebundener Gase bildet sich ein Spektrum von Haftstellen aus, welche die Ladungsträger bei einer Feldstärkenänderung unterschiedlich schnell aufnehmen oder abgeben. Aufgrund dieses sog. Influenzeffektes spiegelt sich dieses komplexe Zeitverhalten auch im Verlauf des Leitwertes wieder, und zwar im Bereich von besonderen Ausschlägen. Folglich lassen sich in einem elektronischen Speicher "Muster" ablegen, die typisch sind für das Altern eines Halbleiter-Gassensors und mit den Ausschlägen und deren zeitlichen Verlauf verglichen werden können.

Im folgenden wird die Erfindung anhand von schematischen Zeichnungen ausführlicher erläutert. Es zeigen
- Fig. 1: ein Schaltschema einer erfindungsgemäßen Einrichtung,
- Fig.2: in gegenüber der Fig. 1 vergrößertem Maßstab den Halbleiter-Gassensor aus dem Gegenstand der Fig. 1,
- Fig. 3: einen Meßschrieb zu Meßergebnissen, die mit einer Einrichtung nach Fig. 1 ermittelt worden sind,
- Fig. 4: ein Schaltschema einer anderen erfindungsgemäßen Einrichtung,
- Fig. 5: in gegenüber der Fig. 4 vergrößertem Maßstab den Halbleiter-Gassensor aus dem Gegenstand der Fig. 4,
- Fig. 6 bis 9: zur Erläuterung der Funktionsweise des Gegenstandes der Fig. 1 und 4 aufgenommene Meßschriebe.

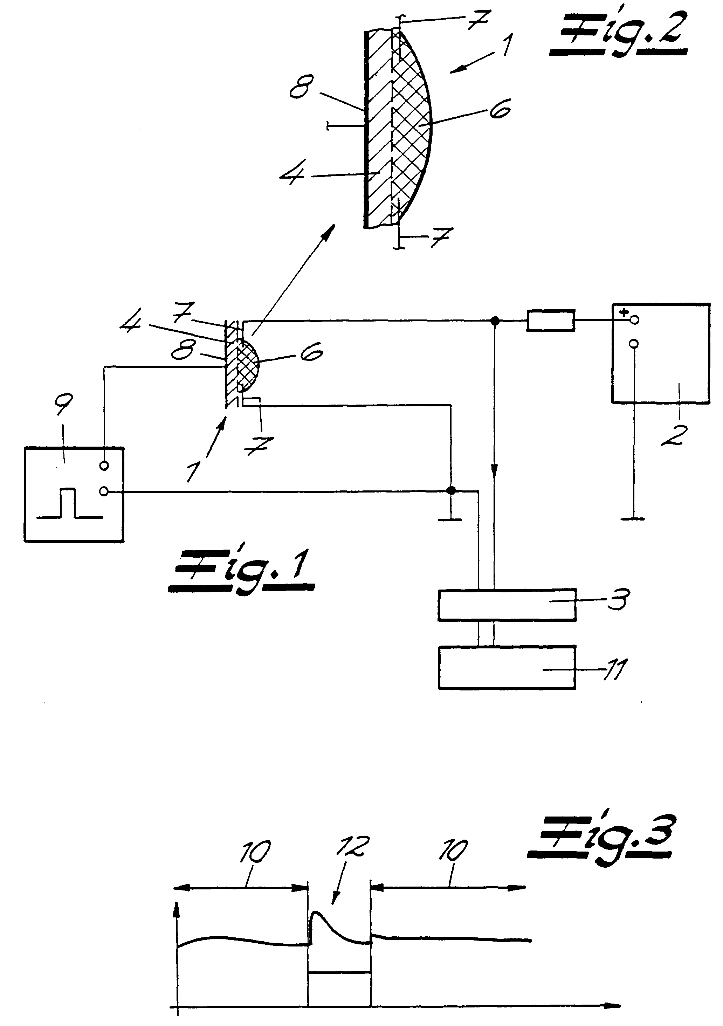

Die in den Fig. 1 und 2 sowie 4 und 5 dargestellte Einrichtung dient zur Messung von Zustandsgrößen in Gasen und weist zumindest einen Halbleiter-Gassenor 1 auf. Der Halbleiter-Gassensor 1 befindet sich in einer Schaltungsanordnung mit einem Netzteil 2 und einem Meßsignalaufnehmer 3. Der Halbleiter-Gassensor 1 weist eine Isolierschicht 4 und auf der Isolierschicht 4 eine Feldelektrode 8 und zwei mit Abstand voneinander angeordneten Elektroden 7 auf. Die beiden Elektroden 7 sind in eine aktive Halbleiterschicht 6 eingebettet, die Feldelektrode 8 ist gegen die Halbleiterschicht 6 und gegen die Elektroden 7 isoliert. Ein Pulsspannungs-Generator 9 ist einerseits an die Feldelektrode 8 und andererseits in die in die Halbleiterschicht 6 eingebetteten Elektroden 7 angeschlossen.

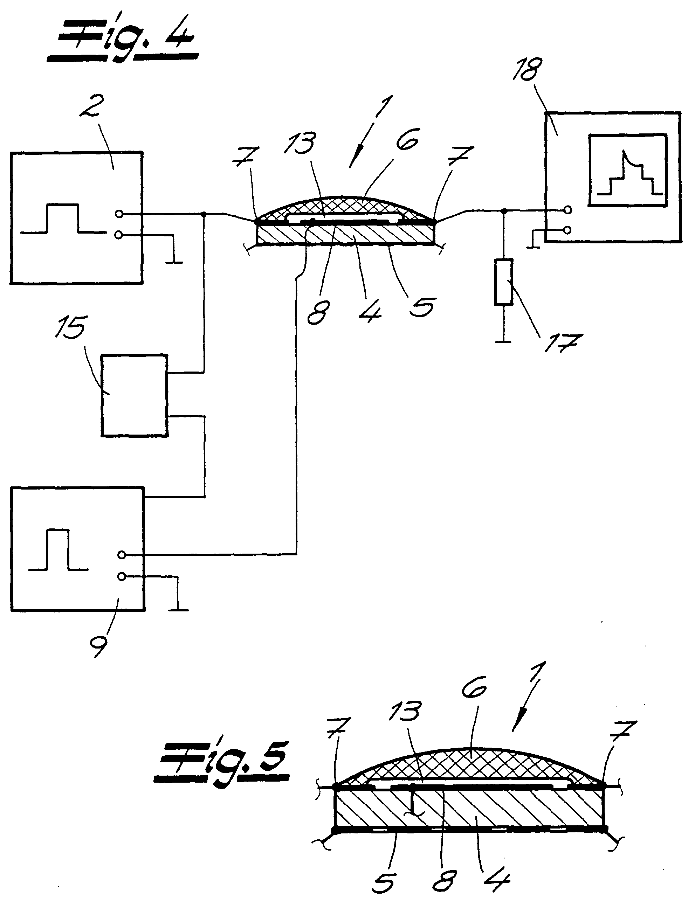

Bei der Ausführungsform nach den Fig. 1 und 2, zu der der Meßschrieb der Fig. 3 gehört, ist die Anordnung so getroffen, daß der Halbleiter-Gassensor 1 auf einer Seite der Isolierschicht 4 die beiden Elektroden 7 und die aktive Halbleiterschicht 6 und auf der anderen Seite der Isolierschicht 4 die Feldelektrode 8 aufweist.

Bei der Durchführung der Messung von Zustandsgrößen in Gasen befindet sich bei der Ausführungsform nach den Fig. 1 und 2 der Halbleiter-Gassensor in dem Gas. Zur Messung der Zustandsgrößen wird der Leitwert der aktiven Halbleiterschicht 6 mit Hilfe der beiden Elektroden 7 gemessen. Die Fig. 3 zeigt den Leitwertverlauf über der Zeit in den Bereichen 10. Der Meßschrieb ist mit Hilfe eines entsprechenden, als Auswerter arbeitenden Microcontrollers 11 ermittelt oder errechnet worden. Außerdem ist zur autogenen Funktionskontrolle des Halbleiter-Gassensors 1 in zeitlichen Abständen ein Puls des Pulsspannungs-Generators 9 zwischen Feldelektrode 8 und einem der in die aktive Halbleiterschicht 6 eingebetteten Elektroden 7 anlegbar. Daraus resultiert eine Leitwertveränderung, die sich in dem Meßschrieb der Fig. 3 im Ausführungsbeispiel als positiver Ausschlag 12 darstellt, der vergleichsweise langsam abklingt. Dieser Ausschlag 12 enthält eine Information über den Alterungszustand des Halbleiter-Gassensors, wie weiter unten ausführlicher ausläutert wird. Der Ausschlag 12 und sein Abklingen können mit einem Grundmuster verglichen werden.

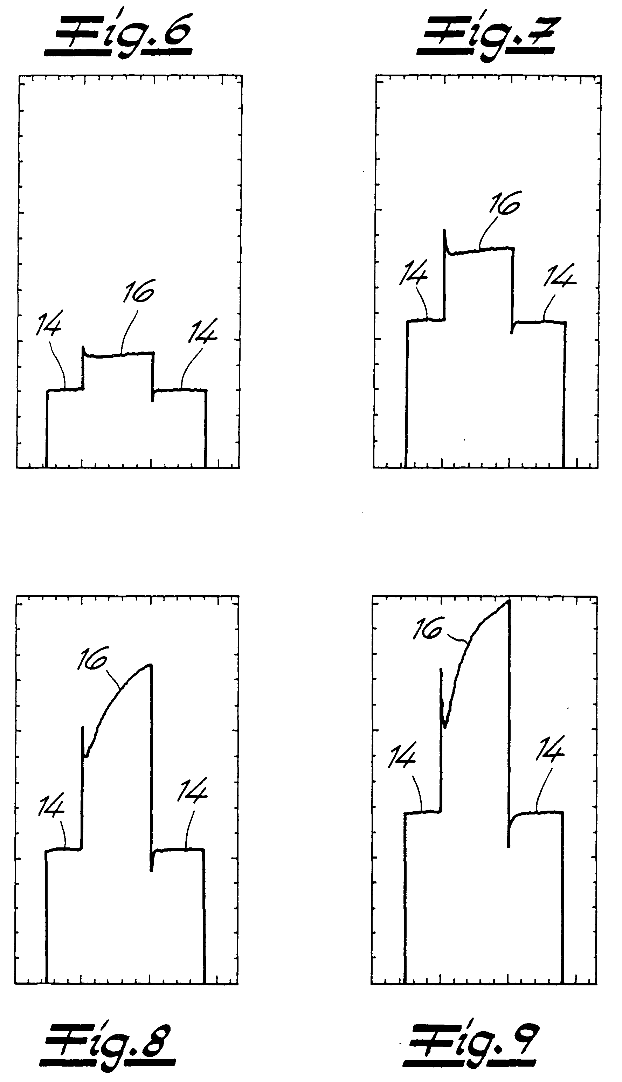

Bei der Ausführungsform nach den Fig. 4 und 5 weist der Halbleiter-Gassensor 1 auf der im Bild oberen Seite der Isolierschicht 4 die beiden Elektroden 7 und, getrennt von diesen, die Feldelektrode 8 auf, die durch eine abdeckende Glasisolierschicht 13 von der aktiven Halbleiterschicht 6, in die die Elektroden 7 eingebettet sind, und damit auch von den Elektroden 7 getrennt ist. Hier ist eine Leiterstruktur 5 vorgesehen, die an einen Heizkreis angeschlossen ist. Sie befindet sich auf der Unterseite der Isolierschicht 4.

Die Betriebsweise für die Ausführungsform einer erfindungsgemäßen Einrichtung nach den Fig. 4 und 5 wird beispielhaft anhand der Fig. 6 bis 9 erläutert. Der Halbleiter-Gassensor 1 wird auf eine konstante Temperatur geheizt. Die Betriebsspannung zur Messung des Stromes durch den Halbleiter-Gassensor 1 wird mit Hilfe eines mit einem Pulsgenerator ausgerüsteten Netzteils 2 periodisch angelegt. Insoweit wird im folgenden von Meßpulsen 14 gesprochen. Mit einer Verzögerung von 5 ms nach Einschalten der Betriebsspannung wird mit Hilfe eines Delay-Bausteines 15 ein Spannungspuls der Dauer von 10 ms angelegt. Insoweit wird von einem Feldpuls 16 gesprochen. Er wird über einen triggerbaren Pulsgenerator 9 mit einer Spannung von 50 V angelegt. Dadurch wird ein Feld mit einer Feldstärke von ca. 5 x 10³ V/cm erzeugt. Die Höhe des Stromes durch die aktive Halbleiterschicht 6 wird als Spannung über einen in Serie geschalteten Widerstand 17, der mindestens um eine Größenordnung kleiner ist als der Widerstand der Halbleiterschicht 6, abgegriffen. Die zugehörige Schaltung erkennt man in der Fig. 4. - Die zu messende Zustandsgröße sei der Gehalt eines Luftstromes an Kohlenmonoxid.

Betrachtet man die zeitliche Abhängigkeit des Stromes während eines Meßpulses 14 und während des überlagerten Feldpulses 16 in einem Oszillographen 18, so ergeben sich die in den Fig. 6 bis 9 gezeichneten Kurven. Die Fig. 6 zeigt die Stromantwort auf die Pulse 14, 16 bei einem frisch präparierten Halbleiter-Gassensor 1 nach der Lehre der Erfindung in Luft und ohne Anwesenheit von Kohlenmonoxid in der Luft. Insoweit wird hier und im folgenden kurz von Strompulsen 14 und 16 gesprochen. Die Fig. 7 zeigt die Stromantwort bei dem gleichen, frisch präparierten Halbleiter-Gassensor 1 bei einem Angebot von 20 ppm Kohlenmonoxid. Im Vergleich zu Fig. 3 hat sich der zeitliche Verlauf des Stromes nur wenig verändert. Die Stromstärke der Stromantwort im Strompuls 14 hat jedoch, dem niedrigeren Widerstand des Halbleiter-Gassensors 1 bei Anwesenheit von Kohlenmonoxid entsprechend, zugenommen. Fig. 8 zeigt die Stromantwort bei dem gleichen Halbleiter-Gassensor 1 nach Alterung, gemessen in Luft ohne Anwesenheit von Kohlenmonoxid. Im Vergleich mit Fig. 6 fällt die größere Höhe des Strompulses 14 auf. Das bedeutet, daß der Widerstand der Halbleiterschicht 6 durch die Alterung abgenommen hat. Bei einer Auswertung ohne Korrektur, beruhend lediglich auf dem Widerstandswert des Halbleiter-Gassensors 1, würde hier fälschlicherweise eine Anzeige erfolgen, die als Anwesenheit von Kohlenmonoxid gedeutet würde. Vergleicht man zusätzlich den zeitlichen Verlauf der Kurve des Strompulses 16, so unterscheidet sich dieser in der Fig. 8 deutlich von der in Fig. 6. Dieser Unterschied, insbes. der Anstieg des Stromes im Strompuls 16 einige Millisekunden nach Einschalten, ist zur Erkennung der Alterung dieses Halbleiter-Gassensors 1 auswertbar.

Die Fig. 9 zeigt die Stromantwort des gealterten Halbleiter-Gassensors 1 während des Angebotes von 20 ppm Kohlenmonoxid. Auch hier ist die im Vergleich zur korrespondierenden Fig. 7 deutlich geänderte Form des Strompulses 16 zu erkennen. Die Veränderung ist abtrennbar, so daß die Auswertung der Pulsform zur Alterurgserkennung unabhängig von der Kohlenmonoxid-Konzentraticn erfolgen kann.

## Patentansprüche

1. Einrichtung für die Messung von Zustandsgrößen in Gasen mit zumindest einem Halbleiter-Gassensor (1) in einer Schaltungsanordnung mit Netzteil (2) und Meßsignalaufnehmer (3) - mit den folgenden Merkmalen:
a) der Halbleiter-Gassensor (1) weist zwei mit Abstand voneinander angeordnete Elektroden (7) sowie eine Isolierschicht (4) und auf der Isolierschicht (4) eine Feldelektrode (8) auf,
b) die beiden Elektroden (7) sind in eine sensor aktive Halbleiterschicht (6) eingebettet, die Feldelektrode (8) ist gegen die Halbleiterschicht (6) und gegen die Elektroden elektrisch isoliert,
c) ein Pulsspannungs-Generator (9) ist einerseits an die Feldelektrode (8) und andererseits an eine der in die Halbleiterschicht (6) eingebetteten Elektroden (7) angeschlossen,
wobei zur Messung der Zustandsgrößen der Leitwert (10) der aktiven Halbleiterschicht (6) über die Elektroden (7) gemessen wird und außerdem zur autogenen Funktionskontrolle des Halbleiter-Gassensors in zeitlichen Abständen ein Puls des Pulsspannungs-Generators (9) zwischen Feldelektrode (8) und eine der in die aktive Halbleiterschicht (6) eingebetteten Elektroden (7) angelegt wird und die durch das vom Spannungspuls erzeugte elektrische Feld verursachte Leitwertänderung (12) der Halbleiterschicht (6) als Funktion der Zeit über den Meßsignalaufnehmer (3) gemessen wird.

2. Einrichtung nach Anspruch 1, wobei der Halbleiter-Gassensor (1) auf einer Seite der Isolierschicht (4) die beiden Elektroden (7) sowie die aktive Halbleiterschicht (6) und auf der anderen Seite der Isolierschicht (4) die Feldelektrode (8) aufweist.

3. Einrichtung nach Anspruch 1, wobei der Halbleiter-Gassensor (1) auf einer Seite der Isolierschicht (4) die beiden Elektroden (7) und, getrennt von diesen, die Feldelektrode (8) aufweist, die durch eine abdeckende Glasisolierschicht (13) von der aktiven Halbleiterschicht (6), in die die Elektroden (7) eingebettet sind, getrennt ist.

4. Einrichtung nach einen der Ansprüche 1 bis 3, wobei zusätzlich auf der Isolierschicht (4) eine Leiterstruktur (5) angeordnet ist, die einem Heizkreis angehört.

5. Einrichtung nach einem der Ansprüche 1 bis 4, wobei als Isolierschicht eine Dünnschicht mit einer Dicke von 30 bis 100 nm eingesetzt ist.

6. Einrichtung nach einem der Ansprüche 1 bis 5, wobei die Isolierschicht aus Siliziumoxid und/oder Siliziumnitrid und/oder aus Aluminiumoxid besteht.

7. Einrichtung nach einem der Ansprüche 4 bis 6, wobei die Leiterstruktur aus einem gut leitenden Metall oder aus dotiertem Polysilizium besteht.

8. Einrichtung nach einem der Ansprüche 1 bis 7, wobei die Auslegung so getroffen ist, daß die Feldelektrode elektrische Feldstärken in der Größenordnung von 10⁴ bis 10⁶ V/cm erzeugt.

9. Einrichtung nach einem der Ansprüche 1 bis 8, wobei das Netzteil (2) für eine Spannung von etwa 10 V ausgelegt ist.

10. Einrichtung nach einem der Ansprüche 1 bis 9, wobei die Schaltungsanordnung einen Microcontroller aufweist und dieser den Ablauf der Meßvorgänge steuert sowie außerdem sowohl bei der Leitwertmessung im Rahmen der Messung der Zustandsgrößen als auch bei der autogenen Funktionskontrolle des Halbleiter-Gassensors als Auswerter eingesetzt ist.

11. Einrichtung nach einem der Ansprüche 4 bis 10, wobei zur Einstellung von kritischen Meßbereichen des aktiven Halbleiter-Gassensors dessen Betriebstemperatur über die Leiterstruktur, an die ein Heizkreis angeschlossen ist, veränderbar ist.

12. Einrichtung nach einem der Ansprüche 1 bis 11, wobei der Betrieb des aktiven Halbleiter-Gassensors mit Gleichspannung oder mit periodisch angelegter Gleichspannung erfolgt.

## Claims

1. A device for measuring state quantities in gases, comprising at least one semiconductor gas sensor (1) in a circuit arrangement comprising a power supply (2) and a measuring signal sensing element (3) - having the following features:
a) the semiconductor gas sensor (1) comprises two electrodes (7) disposed at a spacing from each other, and comprises an insulating layer (4) and a field electrode (8) on the insulating layer (4),
b) the two electrodes (7) are embedded in a sensor-active semiconductor layer (6), and the field electrode (8) is electrically insulated from the semiconductor layer (6) and from the electrodes,
c) one side of a pulsed voltage generator (9) is connected to the field electrode (8) and the other side of said pulsed voltage generator is connected to one of the electrodes (7) which are embedded in the semiconductor layer (6),
wherein in order to measure state quantities the electrical conductance (10) of the active semiconductor layer (6) is measured via the electrodes (7), and in addition a pulse from the pulsed voltage generator (9) is applied at chronological intervals between the field electrode (8) and one of the electrodes (7) embedded in the active semiconductor layer (6) for the self-generated operational monitoring of the semiconductor gas sensor, and the change in electrical conductance (12) of the semiconductor layer (5) which is caused by the electrical field generated by the voltage pulse is measured as a function of time via the measuring signal sensing element (3).

2. A device according to claim 1, wherein the semiconductor gas sensor (1) comprises the two electrodes (7) as well as the active semiconductor layer (6) on one side of the insulating layer (4), and comprises the field electrode (8) on the other side of the insulating layer (4).

3. A device according to claim 1, wherein the semiconductor gas sensor (1) comprises, on one side of the insulating layer (4), the two electrodes (7) and, separated therefrom, the field electrode (8), which is separated from the active semiconductor layer (6) in which the electrodes (7) are embedded by a covering glass insulating layer (13).

4. A device according to any one of claims 1 to 3, wherein a conductor structure (5), which forms part of a heater circuit, is additionally disposed on the insulating layer (4).

5. A device according to any one of claims 1 to 4, wherein a thin film having a thickness of 30 to 100 nm is used as an insulating layer.

6. A device according to any one of claims 1 to 5, wherein the insulating layer consists of silica and/or of silicon nitride and/or of alumina.

7. A device according to any one of claims 4 to 6, wherein the conductor structure consists of a metal having a good conductivity or of doped polysilicon

8. A device according to any one of claims 1 to 7, wherein the design is effected so that the field electrode generates electric field strengths of the order of 10⁴ to 10⁶ V/cm.

9. A device according to any one of claims 1 to 8, wherein the power supply (2) is designed for a voltage of about 10 V.

10. A device according to any one of claims 1 to 9, wherein the circuit arrangement comprises a microcontroller and the latter controls the course of the measuring operations, and is used in addition as an evaluation device, both during the measurement of the electrical conductance in the context of measuring state quantities and during the self-generated operational monitoring of the semiconductor gas sensor.

11. A device according to any one of claims 4 to 10, wherein in order to set critical measuring ranges of the active semiconductor gas sensor, the operating temperature thereof can be varied via the conductor structure, to which a heater circuit is connected.

12. A device according to any one of claims 1 to 11, wherein the active semiconductor gas sensor is operated using a DC voltage or using a periodically applied DC voltage.

## Revendications

1. Dispositif pour la mesure de grandeurs d'état de gaz comportant au moins un capteur de gaz à semi-conducteurs (1) dans un circuit avec bloc secteur (2) et capteur de signaux de mesure (3), présentant les caractéristiques suivantes :
a) le capteur de gaz à semi-conducteurs (1) comporte deux électrodes (7) disposées à distance l'une de l'autre ainsi qu'une couche isolante (4) et sur la couche isolante (4), une électrode de champ (8),
b) les deux électrodes (7) sont noyées dans une couche semi-conductrice (6) active du capteur, l'électrode de champ (8) est isolée électriquement par rapport à la couche semi-conductrice (6) et par rapport aux électrodes,
c) un générateur de tension pulsée (9) est raccordé d'une part à l'électrode de champ (8) et d'autre part à l'une des électrodes (7) noyées dans la couche semi-conductrice (6),
dans lequel pour la mesure des grandeurs d'état, la conductivité (10) de la couche semi-conductrice active (6) est mesurée par les électrodes (7), et en outre, pour le contrôle autogène du fonctionnement du capteur de gaz à semi-conducteurs, une impulsion du générateur de tension pulsée (9) est appliquée, à intervalles de temps, entre l'électrode de champ (8) et une des électrodes (7) noyées dans la couche semi-conductrice active (6), et la variation (12) de la conductivité de la couche semi-conductrice (6), provoquée par le champ électrique généré par l'impulsion de tension, est mesurée en tant que fonction du temps par l'intermédiaire du capteur de signaux de mesure (3).

2. Dispositif selon la revendication 1, dans lequel le capteur de gaz à semi-conducteurs () comporte, sur un côté de la couche isolante (4), les deux électrodes (7) ainsi que la couche semi-conductrice active (6) et, sur l'autre côté de la couche isolante (4), l'électrode de champ (8).

3. Dispositif selon la revendication 1, dans lequel le capteur de gaz à semi-conducteurs (1) comporte, sur un côté de la couche isolante (4), les deux électrodes (7) et, séparée de celles-ci, l'électrode de champ (8) qui est séparée par une couche isolante de verre (13) de la couche semi-conductrice active (6) dans laquelle sont noyées les électrodes (7).

4. Dispositif selon l'une des revendications 1 à 3, dans lequel une structure conductrice (5), qui fait partie d'un circuit de chauffage, est disposée en outre sur la couche isolante (4).

5. Dispositif selon l'une des revendications 1 à 4, dans lequel une couche mince d'une épaisseur de 30 à 100 nm est utilisée comme couche isolante.

6. Dispositif selon l'une des revendications 1 à 5, dans lequel la couche isolante est constituée d'oxyde de silicium et/ou de nitrure de silicium et/ou d'oxyde d'aluminium.

7. Dispositif selon l'une des revendications 4 à 6, dans lequel la structure conductrice est constituée d'un métal bon conducteur ou de polysilicium dopé.

8. Dispositif selon l'une des revendications 1 à 7, dans lequel le dimensionnement est tel que l'électrode de champ produit des intensités de champ électrique d'un ordre de grandeur de 10⁴ à 10⁵ V/cm.

9. Dispositif selon l'une des revendications 1 à 8, dans lequel le bloc secteur (2) est dimensionné pour une tension d'environ 10 V.

10. Dispositif selon l'une des revendications 1 à 9, dans lequel le circuit comporte un microcontrôleur et celui-ci commande le déroulement des opérations de mesure, et est utilisé en outre comme dispositif d'exploitation, aussi bien pour la mesure de la conductivité, dans le cadre de la mesure des grandeurs d'état, que pour le contrôle autogène du fonctionnement du capteur de gaz à semi-corducteurs.

11. Dispositif selon l'une des revendications 4 à 10, dans lequel pour le réglage de plages de mesure critiques du capteur de gaz à semi-conducteurs actif, sa température de service peut être modifiée par la structure conductrice à laquelle est raccordé un circuit de chauffage

12. Dispositif selon l'une des revendications 1 à 11, dans lequel le fonctionnement du capteur de gaz à semi-conducteurs actif s'effectue avec une tension continue ou avec une tension continue appliquée périodiquement.
